(19) Europäisches Patentamt / European Patent Office / Office européen des brevets

(11) **EP 2 966 435 A1**

(12) **EUROPEAN PATENT APPLICATION**
published in accordance with Art. 153(4) EPC

(43) Date of publication:
**13.01.2016 Bulletin 2016/02**

(51) Int Cl.:
*G01N 21/64* (2006.01)   *C08J 3/14* (2006.01)
*C08J 3/20* (2006.01)   *C08L 101/00* (2006.01)
*C09B 67/02* (2006.01)

(21) Application number: **14761005.9**

(22) Date of filing: **04.03.2014**

(86) International application number:
**PCT/JP2014/055469**

(87) International publication number:
**WO 2014/136776 (12.09.2014 Gazette 2014/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **08.03.2013  JP 2013047259**

(71) Applicant: **Konica Minolta, Inc.**
**Tokyo 100-7015 (JP)**

(72) Inventors:
• **TAKAHASHI, Masaru**
  **Tokyo 100-7015 (JP)**
• **TAKANASHI, Kensaku**
  **Tokyo 100-7015 (JP)**

(74) Representative: **Gille Hrabal**
**Brucknerstrasse 20**
**40593 Düsseldorf (DE)**

(54) **RESIN PARTICLES FOR FLUORESCENT LABELS**

(57)     An object of the present invention is to provide a fluorophore having excellent initial emission intensity and light resistance, which is not easily discolored and does not affect the evaluation of the number of bright spots even when it is slightly discolored. The resin particle for fluorescence labeling is characterized by comprising a fluorescent dye immobilized in a resin particle, the fluorescent dye satisfying both of the following conditions (1) and (2): (1) the fluorescent dye alone in an aqueous solution has a concentration-dependent peak emission intensity in a range of 30 to 80 $\mu$M; and (2) the emission intensity at a concentration of 100 $\mu$M is not less than 80% of the peak emission intensity. It is preferred that the Stoke's shift of the fluorescent dye in an aqueous solution is not less than 25 nm. The fluorescent dye may be subjected to a solubilization treatment (such as an acid treatment). It is preferred that the fluorescent dye be encapsulated in a resin particle comprising a thermosetting resin.

[Fig. 1]

EP 2 966 435 A1

**Description**

TECHNICAL FIELD

The present invention relates to a resin particle for fluorescent labeling that can be used in pathological diagnosis and the like.

BACKGROUND ART

As a medical diagnosis, pathological diagnosis is performed. A pathologist diagnoses a disease using a tissue section collected from a human body and informs a clinician of whether or not a therapy and/or surgery is/are necessary. Based on the patient conditions and the pathological diagnosis, a physician determines the pharmacotherapeutic strategies and a surgeon determines whether or not a surgery should be performed.

In pathological diagnosis, it is common practice to prepare a tissue specimen by slicing a tissue sample obtained by evisceration or needle biopsy into a thickness of about several micrometers and then observe the tissue specimen at a magnification under a light microscope so as to obtain various findings. In many cases, a specimen is prepared by fixing a collected tissue through dehydration and paraffin blocking, slicing the thus fixed tissue into a thickness of several micrometers and then removing the paraffin.

In such pathological diagnosis, in order to observe the expression state of a molecule associated with a specific disease (antigen) in a specimen, immunostaining which utilizes immunoreaction between the molecule and a complex composed of an antibody for the molecule and a label is performed. In immunostaining, conventionally, a dye staining method which uses an enzyme reacting with a specific substrate to exhibit a color as a label is employed. For example, DAB staining is a dye staining method which uses, as an enzyme label, peroxidase that has diaminobenzidine (DAB) as a substrate. However, in staining with an enzyme label such as DAB staining, since the staining concentration is largely variable depending on the environmental conditions such as temperature and time, there is a problem that estimation of the actual expression amount of an antibody based on the staining concentration is difficult. Therefore, in recent years, a fluorescent labeling method in which a fluorescent label having excellent quantitative properties is used in place of an enzyme label has been increasingly applied to immunological observation in pathological diagnosis.

Fluorescent dyes, inorganic nanoparticles (which may also be referred to as "semiconductor nanoparticles", "quantum dots" or the like) and aggregates thereof are known to be utilized as fluorescent labels. For example, in Patent Document 1, a fluorescent particle comprising one or more organic dyes and organic fluorescent dyes (donor dye and acceptor dye of FRET) having a longer excitation wavelength than the organic dyes, in which silica is used as a main component of a solid particle (parental material) entrapping a dye molecule, is disclosed. Patent Document 2 discloses silica nanoparticles comprising a fluorescent organic dye (Texas Red is used in Examples), which are used in a pathological diagnosis information generation method (system). Further, Patent Document 3 discloses melamine-formaldehyde particles comprising one or more hydrophilic organic dyes or organic fluorescent dyes (sodium 8-hydroxy-1,3,6-pyrenetrisulfonate is used in Examples).

PRIOR ART REFERENCES

PATENT DOCUMENTS

[Patent Document 1] JP-A-2009-300334
[Patent Document 2] JP-A-2012-208106
[Patent Document 3] Japanese Translated PCT Patent Application Laid-open No. 2008-543982

SUMMARY OF THE INVENTION

PROBLEMS TO BE SOLVED BY THE INVENTION

In a fluorescent labeling method where fluorescence observation is performed under a microscope, it is generally known that excitation light condensed by a lens is likely to cause a fluorophore to be discolored, and there is thus a problem that quantification of the fluorophore using its fluorescence intensity as an index is difficult. Moreover, when the number of bright spots of the fluorophore is used as an index, a large reduction in the emission intensity due to discoloration leads to a reduction in the number of bright spots, which deteriorates the quantitative performance. These are largely problematic particularly in pathological diagnosis.

Fluorophores using silica as a parental material, such as those disclosed in Patent Documents 1 and 2, generally show weak fluorescence intensity and have low light resistance. Further, the organic fluorescent dye-containing particles using the organic fluorescent dye concretely described in Patent Document 3, which use a melamine-formaldehyde (melamine resin) as a parental material, also have room for improvement in terms of the fluorescence intensity and light resistance. Here, sodium 8-hydroxy-1,3,6-pyrenetrisulfonate used in the Examples of the Patent Document 3 is not a fluorescent dye that satisfies the below-described specific standards of the present invention.

An object of the present invention is to provide a fluorophore having excellent initial emission intensity and light resistance, which is not easily discolored and does not affect the evaluation of the number of bright spots even when it is slightly discolored.

TECHNICAL SOLUTION

The present inventors discovered that a resin particle for fluorescent labeling, which is capable of solving the above-described problems, can be prepared by selecting, as a fluorescent dye to be immobilized in a resin particle, one which satisfies specific standards relating to the concentration and emission intensity of an aqueous solution thereof, thereby completing the present invention. That is, such a resin particle for fluorescent labeling - which is obtained by a production method where a fluorescent dye unlikely to cause concentration quenching is selected in accordance with the specific standards, the fluorescent dye is typically mixed with a resin material to allow a resin synthesis reaction to proceed and the fluorescent dye is thereby encapsulated in the resulting resin particle - comprises a relatively large amount of the fluorescent dye being immobilized (encapsulated) but does not cause concentration quenching; therefore, the particle is unlikely to be discolored even if it is irradiated with fluorescent light for a certain period during fluorescence observation. The present invention that was completed based on these discoveries encompasses the following matters.

[1] A resin particle for fluorescent labeling, comprising a fluorescent dye immobilized in a resin particle, the fluorescent dye satisfying both of the following conditions (1) and (2):

(1) the fluorescent dye alone in an aqueous solution has a concentration-dependent peak emission intensity in a range of 30 to 80 $\mu$M (M: molar concentration, mol/L); and
(2) the emission intensity at a concentration of 100 $\mu$M is not less than 80% of the peak emission intensity.

[2] The resin particle for fluorescent labeling according to [1], wherein the Stoke's shift of the fluorescent dye in an aqueous solution is not less than 25 nm.
[3] The resin particle for fluorescent labeling according to [1] or [2], wherein the fluorescent dye has been subjected to a solubilization treatment.
[4] The resin particle for fluorescent labeling according to [3], wherein the solubilization treatment is an acid treatment.
[5] The resin particle for fluorescent labeling according to any one of [1] to [4], wherein the fluorescent dye in an aqueous solution has a peak emission intensity of not less than 100,000 in terms of the brightness represented by the following formula:

```
Brightness = Molar extinction coefficient × Quantum yield

× 1/1,000.
```

[6] The resin particle for fluorescent labeling according to any one of [1] to [5], wherein the fluorescent dye is encapsulated in a resin particle comprising a thermosetting resin.
[7] The resin particle for fluorescent labeling according to any one of [1] to [6], wherein a biologically-relevant binding substance is ligated to the particle surface.

ADVANTAGEOUS EFFECTS OF INVENTION

The resin particle for fluorescent labeling according to the present invention has superior light resistance and is thus less likely to be discolored as compared to conventional resin particles; therefore, reduction in the fluorescence intensity with observation time (irradiation of excitation light) can be inhibited. In addition, the resin particle for fluorescent labeling according to the present invention also satisfies a high level of initial emission intensity. These features consequently make it possible to measure bright spots while maintaining the quantitative performance without being affected by the observation time. This effect is useful particularly for improving the accuracy of pathological diagnosis.

BRIEF DESCRIPTION OF DRAWINGS

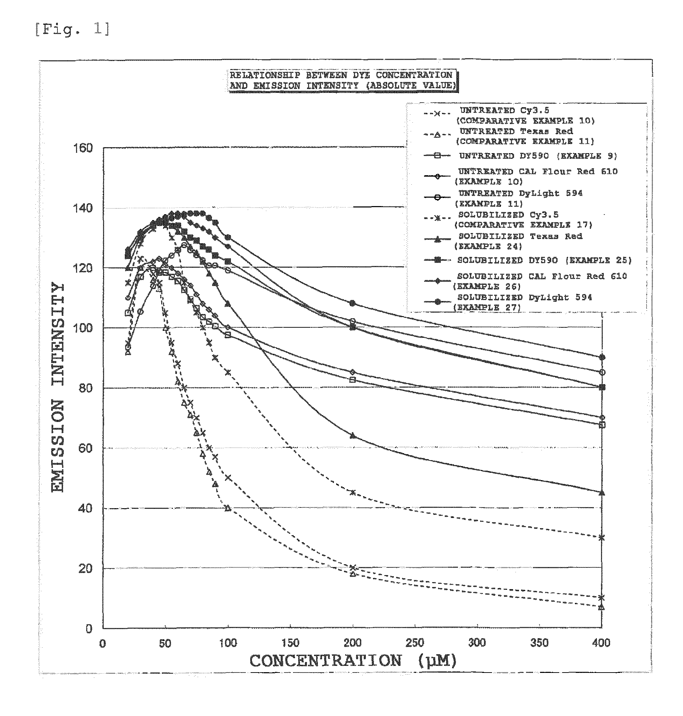

[Fig. 1] Fig. 1 is a graph showing the relationship between the aqueous solution concentration and the emission intensity for those cases where five representative fluorescent dyes used in Examples and Comparative Examples were or were not each subjected to a solubilization treatment.

MODE FOR CARRYING OUT THE INVENTION

-Resin Particle for fluorescent labeling-

The term "resin particle for fluorescent labeling" used herein is a general term for a substance having a structure in which plural fluorescent dye molecules are immobilized in a resin particle by chemical or physical action, and its form is not particularly restricted.

Examples of the resin particle for fluorescent labeling include those in a form that a fluorescent dye is mainly encapsulated in a resin particle (encapsulation-type), which are obtained by a production method where polymerization reaction of a resin material such as a melamine resin is allowed to proceed while mixing the resin material with a fluorescent dye. Such encapsulation-type resin particles for fluorescent labeling are preferred because a large number of fluorescent dye molecules can be immobilized thereon and, according to the present invention, high fluorescence intensity and high light resistance can be achieved by selecting an appropriate fluorescent dye and thereby suppressing concentration quenching. Thereamong, a resin particle for fluorescent labeling in which a fluorescent dye is encapsulated in a resin particle that contains a thermosetting resin forming a fine cross-linked structure (e.g., a resin particle composed of a melamine resin) is particularly preferred.

Meanwhile, examples of the resin particle for fluorescent labeling also include those in a form that a fluorescent dye is mainly adsorbed on the surface of a resin particle (adsorption-type), which are obtained by a production method where resin particles are prepared using a resin material such as a melamine resin and a fluorescent dye is subsequently added to a dispersion of the resin particles.

In the case of an encapsulation-type resin particle for fluorescent labeling, the fluorescent dye is confined inside the particle due to at least the molecular structure of the resin (for example, a three-dimensional network structure of a thermosetting resin such as a melamine resin). Further, it is preferred that the fluorescent dye be more strongly immobilized in the resin particle by the work of electrostatic interaction (ionic bond) between a functional group or moiety of the fluorescent dye and that of the resin or by a covalent bond formed therebetween, because this enables to inhibit elution of the fluorescent dye out of the particle, which is caused by an organic solvent (xylene) used in the clearing step of tissue staining.

The above-described "electrostatic interaction" is exerted when the fluorescent dye has a positively or negatively charged substituent or moiety and the resin molecule has a substituent or moiety with an opposite charge. The phrase used herein, "has a positively or negatively charged substituent or moiety", means that the fluorescent dye or resin can be in such a state when dissolved in neutral, acidic or basic water. Representative examples of the negatively charged substituent or moiety include a sulfo group ($-SO_3^-$) as well as a phosphoryl group, a phenolic hydroxyl group and a carboxyl group. Representative examples of the positively charged substituent or moiety include an amino group ($-NH_3^+$) as well as aromatic amino groups, cyclic amino groups (e.g., a pyridinyl group and a triazolyl group) and a hydrazinyl group. As for the above-described substituent or moiety of the resin, it may be introduced to the fluorescent dye and the resin material (monomer) in advance to the production process of the resin particle for fluorescent labeling, or the substituent or moiety may be formed in the protonation process of amino group contained in a melamine resin, urea resin or the like by an addition of $H^+$ thereto. Further, as for the above-described substituent or moiety of the fluorescent dye, it may be already included in the fluorescent dye molecule as a commercially available product, or the substituent or moiety may be introduced by "solubilization treatment" (acid treatment) in the present invention. The larger the number of the charged substituents or the like contained in the resin and the fluorescent dye (per molecule or per prescribed unit of molecular weight), the stronger tends to become the electrostatic interaction; therefore, it is preferred that the number of the charged substituents or the like be adjusted such that the electrostatic interaction is exerted at a desired strength.

Further, examples of the "covalent bond" include an amide bond, an ester bond, an ether bond and a C-N bond. For example, as the fluorescent dye and the resin material (monomer), ones that comprise a functional group or moiety capable of forming the above-described covalent bond can be selected; a preliminary step of allowing them to react with each other may be arranged prior to the resin synthesis reaction; and then, in the polymerization step, the thus derivatized resin material obtained in the preliminary step can be allowed to react along with other resin material (co-monomer) as required. Alternatively, a mode in which, with or without such a preliminary step, for the unreacted compound, a covalent bond is formed in the polymerization step by reaction between the fluorescent dye added to the resin material and the resin material or generated resin.

Meanwhile, in the case of an adsorption-type resin particle for fluorescent labeling, in order to immobilize a fluorescent dye on the resin particle, it is required that electrostatic interaction be exerted between a functional group or moiety existing on the surface of the resin particle and that of the fluorescent dye, or a covalent bond be formed therebetween. For an adsorption-type resin particle as well, electrostatic interaction can be utilized in the same manner as in the case of the above-described encapsulation-type resin particle. For the formation of a covalent bond, as in the case of the encapsulation-type resin particle, a step of forming a covalent bond can be arranged after the synthesis of the resin particle.

The size of the resin particle for fluorescent labeling is not particularly restricted as long as it is suitable for the intended application such as immunostaining of a tissue section; however, it is usually 10 to 500 nm, preferably 50 to 200 nm. Further, the variation coefficient, which represents the variation in the particle size, is also not particularly restricted; however, it is usually 20% or less, preferably 5 to 15%. A resin particle for fluorescent labeling that has such a particle size can be obtained by, for example, the below-described production method.

The size of a resin particle for fluorescent labeling can be determined by taking an electron micrograph thereof using a scanning electron microscope (SEM), measuring the cross-sectional area of the resin particle for fluorescent labeling and then determining the particle size as the diameter of a circular area corresponding to the measured value (circular area-equivalent diameter). With regard to the average particle size (average particle diameter) and the variation coefficient of a group of the resin particles for fluorescent labeling, after measuring the particle size for a sufficient number (for example, 1,000) of the resin particles in the above-described manner, the average particle size is calculated as the arithmetic mean of the measured values, and the variation coefficient is calculated by the following equation: $100 \times$ (standard deviation of particle size)/(average particle size).

The resin particle for fluorescent labeling according to the present invention can be prepared as a resin particle which shows sufficient initial emission intensity at the initiation of irradiation with an excitation light and is capable of retaining a certain level of emission intensity even after being irradiated with an excitation light for a prescribed time, that is, a resin particle having excellent light resistance. The initial emission intensity and the emission intensity retention rate vary depending on the selection of various conditions and materials relating to the production of the resin particle for fluorescent labeling as well as the evaluation method and the like; therefore, an absolute evaluation standard cannot be set. For instance, when the resin particle for fluorescent labeling according to the present invention is produced and its light resistance (after being irradiated with an excitation light for 10 minutes) is evaluated in such an embodiment as shown in the below-described Examples, the resin particle for fluorescent labeling according to the present invention has an emission intensity retention rate of preferably not less than 60%, more preferably not less than 75%.

(Fluorescent Dye)

In the present invention, the fluorescent dye is not particularly restricted as long as it satisfies both of the following conditions: (1) the fluorescent dye alone in an aqueous solution has a concentration-dependent peak emission intensity in a range of 30 to 80 μM; and (2) the emission intensity at a concentration of 100 μM is not less than 80% of the peak emission intensity.

Whether or not a fluorescent dye satisfies the condition (1) can be judged based on, when multi-step dilutions of an aqueous solution containing only the fluorescent dye are prepared and the fluorescence intensities of the dilutions are measured under the same condition and compared, whether or not the concentration of the dilution showing a peak emission intensity is within a range of 30 to 80 μM.

Whether or not a fluorescent dye satisfies the condition (2) can be judged based on whether or not the emission intensity of a dilution of the fluorescent dye having a concentration of 100 μM, which is prepared for the above-described measurement relating to the condition (1), is 80% or higher of the above-described peak emission intensity.

The condition (2) can also be set such that the fluorescent dye at a concentration of 100 μM has an emission intensity of preferably not less than 85%, more preferably not less than 90%, of the peak emission intensity.

The method of measuring the emission intensity is not particularly restricted, and the emission intensity can be measured using an ordinary fluorophotometer under ordinary measurement conditions. Usually, the wavelength of the excitation light to be irradiated and the emission wavelength to be measured are set to be the maximum excitation wavelength and the maximum emission wavelength of each fluorescent dye, or wavelengths close thereto, respectively. Further, the slit width for the excitation light and light emission can be adjusted within an appropriate range. The maximum excitation wavelength and the maximum emission wavelength can be measured by a conventional method, and these wavelengths of a commercially available fluorescent dye may be assumed to be the ones that are described in the catalogue thereof.

In general, the larger the Stoke's shift of a fluorescent dye, that is, the larger the difference between the maximum excitation wavelength and the maximum emission wavelength, the smaller becomes the overlapping of the absorption spectrum and the emission spectrum (self-absorption) of the fluorescent dye. Consequently, the occurrence of concentration quenching is suppressed, and this makes it easier to obtain a resin particle for fluorescent labeling having a high emission intensity and improves the quantitative sensitivity. In the present invention, as long as the above-described conditions (1) and (2) are satisfied, the size of the Stoke's shift of the fluorescent dye to be used is not particularly restricted. For example, a fluorescent dye whose Stoke's shift is 25 nm or larger is preferred not only because it is likely to satisfy the conditions (1) and (2) but also because it makes it easy to prepare a resin particle for fluorescent labeling that has an emission intensity appropriate for an intended application.

By subjecting a fluorescent dye to a solubilization treatment, the fluorescent dye is made more likely to satisfy the conditions (1) and (2). That is, among those fluorescent dyes that usually do not satisfy the conditions (1) and (2), there are some fluorescent dyes that can be made to satisfy the conditions (1) and (2) by a solubilization treatment and thus be used in the present invention in the same manner as a fluorescent dye that satisfies the conditions (1) and (2) even without a solubilization treatment. It is noted here that such a fluorescent dye that satisfies the conditions (1) and (2) even without a solubilization treatment may also be subjected to a solubilization treatment before being used (usually, the satisfaction of the conditions (1) and (2) is naturally maintained). By a solubilization treatment of the fluorescent dye, particularly in encapsulation-type resin particles for fluorescent labeling, the dispersion and emission intensity of the dye are improved and particles having a high emission intensity and high light resistance can be consequently obtained, so that the quantitative sensitivity in pathological observation can be improved.

Further, by subjecting a fluorescent dye to a solubilization treatment, the Stoke's shift of the fluorescent dye may be increased to 25 nm or larger. Usually, a fluorescent dye whose Stoke's shift is less than 25 nm can be subjected to a solubilization treatment so as to increase the Stoke's shift to 25 nm or larger.

The above-described solubilization treatment is not particularly restricted as long as it is a technique capable of solubilizing a fluorescent dye, that is, improving the solubility of the fluorescent dye in water. Specific examples of the solubilization treatment include methods in which a fluorescent dye is treated and reacted with an acid (e.g., concentrated sulfuric acid, concentrated hydrochloric acid, acetic acid or formic acid) or an aldehyde (e.g., formaldehyde or acetaldehyde). Thereamong, an acid treatment is preferred because of its excellent effect.

The compound preferably used in the solubilization treatment, for example, the acid to be used in the acid treatment, varies depending on the fluorescent dye to be treated. For instance, concentrated hydrochloric acid is suitably used for HiLyte Fluor, whereas the use of concentrated sulfuric acid causes quenching. For Dylight 594, concentrated sulfuric acid is suitable, whereas the use of concentrated hydrochloric acid causes discoloration. Nonetheless, those of ordinary skill in the art should be able to select an appropriate compound from candidate compounds (acids) without having to bear undue trial and error.

In so far as the conditions (1) and (2) are judged, it presents no problem even if the measured emission intensity values are relative numerical values represented in an arbitrary unit (a.u.), as long as the measurement conditions are the same between the subjects to be compared. However, the measured values may also be absolute numerical values such as brightness represented by the following formula.

$$\text{Brightness} = \text{Molar extinction coefficient } (\varepsilon) \times \text{Quantum}$$
$$\text{yield } (\varphi) \times 1/1,000$$

The brightness of the fluorescent dye used in the present invention, which is represented by the above formula, is not particularly restricted as long as a resin particle for fluorescent labeling that has an emission intensity appropriate for an intended application can be produced. For example, the above-described fluorescent dye having a brightness of not less than 100,000 at the peak emission intensity is preferred because such a resin particle for fluorescent labeling is easily produced by using the fluorescent dye.

Fluorescent dyes that are generally available or can be prepared may be classified into, for example, rhodamine-based dye molecules, squarylium-based dye molecules, cyanine-based dye molecules, aromatic hydrocarbon-based dye molecules, oxazine-based dye molecules, carbopyronine-based dye molecules, pyrromethene-based dye molecules, Alexa Fluor (registered trademark, manufactured by Invitrogen)-based dye molecules, BODIPY (registered trademark, manufactured by Invitrogen)-based dye molecules, Cy (registered trademark, manufactured by GE Healthcare)-based dye molecules, DY (registered trademark, manufactured by Dyomics GmbH)-based dye molecules, HiLyte (registered trademark, manufactured by AnaSpec Inc.)-based dye molecules, DyLight (registered trademark, manufactured by Thermo Fisher Scientific K.K.)-based dye molecules, ATTO (registered trademark, manufactured by ATTO-TEC GmbH)-based dye molecules and MFP (registered trademark, manufactured by Mobitec Co., Ltd.)-based dye molecules. The generic names of these dye molecules are designated based on the main structure (skeleton) or registered trademark of the respective compounds; therefore, those of ordinary skill in the art should be able to properly understand the scope of the fluorescent dyes belonging to the respective generic names without having to bear undue trial and error.

Specific examples of the rhodamine-based dye molecules include 5-carboxy-rhodamine, 6-carboxy-rhodamine, 5,6-dicarboxy-rhodamine, rhodamine 6G, tetramethyl rhodamine, X-rhodamine (ROX), 5-carboxy-X-rhodamine (5-ROX), Texas Red, Spectrum Red, LD700 PERCHLORATE, CAL Fluor Red 610 and CAL Fluor Red 615.

Specific examples of the squarylium-based dye molecules include SRfluor 680-carboxylate,

1,3-bis[4-(dimethylamino)-2-hydroxyphenyl]-2,4-dihydroxycyclo butenediylium dihydroxide,
bis,1,3-bis[4-(dimethylamino)phenyl]-2,4-dihydroxycyclobutene diylium dihydroxide,
bis,2-(4-(diethylamino)-2-hydroxyphenyl)-4-(4-(diethyliminio)     -2-hydroxycyclohexa-2,5-dienylidene)-3-oxocyclobut-1-enolate,
2-(4-(dibutylamino)-2-hydroxyphenyl)-4-(4-(dibutyliminio)-2-h   ydroxycyclohexa-2,5-dienylidene)-3-oxocyclobut-1-enolate, and
2-(8-hydroxy-1,1,7,7-tetramethyl-1,2,3,5,6,7-hexahydropyrido[ 3,2,1-ij]quinolin-9-yl)-4-(8-hydroxy-1,1,7,7-tetramethyl-2,3,
6,7-tetrahydro-1H-pyrido[3,2,1-ij]quinolinium-9(5H)-ylidene)-3-oxocyclobut-1-enolate.

Specific examples of the cyanine-based dye molecules include

1-butyl-2-[5-(1-butyl-1,3-dihydro-3,3-dimethyl-2H-indol-2-yli dene)-penta-1,3-dienyl]-3,3-dimethyl-3H-indolium hexafluorophosphate,
1-butyl-2-[5-(1-butyl-3,3-dimethyl-1,3-dihydro-indol-2-yliden     e)-3-chloro-penta-1,3-dienyl]-3,3-dimethyl-3H-indolium hexafluorophosphate, and
3-ethyl-2-[5-(3-ethyl-3H-benzothiazol-2-ylidene)-penta-1,3-di enyl]-benzothiazol-3-ium iodide.

Specific examples of the aromatic hydrocarbon-based dye molecules include

N,N-bis-(2,6-diisopropylphenyl)-1,6,7,12-(4-tert-butylphenoxy )-perylene-3,4,9,10-tetracarbonacid diimide,
N,N'-bis(2,6-diisopropylphenyl)-1,6,7,12-tetraphenoxyperylene -3,4:9,10-tetracarboxdiimide, N,N'-bis(2,6-diisopropylphenyl)perylene-3,4,9,10-bis(dicarbim ide),
16,N,N'-bis(2,6-dimethylphenyl)perylene-3,4,9,10-tetracarboxy 11c diimide,
4,4'-[(8,16-dihydro-8,16-dioxodibenzo[a,j]perylene-2,10-diyl) dioxy]dibutyric acid,
2,10-dihydroxy-dibenzo[a,j]perylene-8,16-dione,
2,10-bis(3-aminopropoxy)dibenzo[a,j]perylene-8,16-dione,
3,3'-[(8,16-dihydro-8,16-dioxodibenzo[a,j]perylene-2,10-diyl) dioxy]dipropylamine,
17-bis(octyloxy)anthra[9,1,2-cde-]benzo[rst]pentaphene-5-10-d ione, octadecanoic acid,
5,10-dihydro-5,10-dioxoanthra[9,1,2-cde]benzo[rst]pentaphene-16,17-diylester, dihydroxydibenzanthrone, benzenesulfonic acid,
4,,4',4'',4'''-[[2,9-bis[2,6-bis(1-methylethyl)phenyl]-1,2,3,8 ,9,10-hexahydro-1,3,8,10-tetraoxoanthra[2,1,9-def:6,5,10-d'e' f']diisoquinoline-5,6,12,13-tetrayl]tetrakis(oxy)]tetrakis-, benzeneethanaminium, and
4,4',4'',4'''-[[2,9-bis[2,6-bis(1-methylethyl)phenyl]-1,2,3,8 ,9,10-hexahydro-1,3,8,10-tetraoxoanthra[2,1,9-def:6,5,10-d'e' f']diisoquinoline-5,6,12,13-tetrayl]tetrakis(oxy)]tetrakis[N, N,N-trimethyl-].

Specific examples of the oxazine-based dye molecules include Cresyl Violet, Oxazine 170, EVOblue 30 and Nile Blue.

Specific examples of the carbopyronine-based dye molecules include CARBOPYRONIN 149.

Specific examples of the dipyrromethene (pyrromethene)-based dye molecules include PYRROMETHENE 650.

Specific examples of the Alexa Fluor-based dye molecules (mainly having a rhodamine skeleton) include Alexa Fluor 555, Alexa Fluor 568, Alexa Fluor 594, Alexa Fluor 610, Alexa Fluor 633, Alexa Fluor 635, Alexa Fluor 647, Alexa Fluor 660, Alexa Fluor 680, Alexa Fluor 700 and Alexa Fluor 750 (all of which are manufactured by Invitrogen).

Specific examples of the BODIPY-based dye molecules (mainly having a dipyrromethene skeleton) include BODIPY FL, BODIPY TMR, BODIPY TR,BODIPY 493/503, BODIPY 530/550, BODIPY 558/568, BODIPY 564/570, BODIPY 576/589, BODIPY 581/591, BODIPY 630/650 and BODIPY 650/665 (all of which are manufactured by Invitrogen).

Specific examples of the Cy-based dye molecules include Cy 3.5, Cy 5 and Cy 5.5 (all of which are manufactured by GE Healthcare).

Specific examples of the DY-based dye molecules (mainly having a rhodamine skeleton) include DY-590, DY-610, DY-615, DY-630, DY-631, DY-632, DY-633 and DY-634 (all of which are manufactured by Dyomics GmbH).

Specific examples of the HiLyte-based dye molecules include HiLyte 594 and HiLyteFluor TR (both of which are manufactured by AnaSpec Inc.).

Specific examples of the DyLight-based dye molecules (mainly having a rhodamine skeleton) include DyLight 594 and DyLight 633 (both of which are manufactured by Thermo Fisher Scientific K.K.).

Specific examples of the ATTO-based dye molecules (mainly having a rhodamine skeleton) include ATTO 590, ATTO 610, ATTO 620, ATTO 633 and ATTO 655 (all of which are manufactured by ATTO-TEC GmbH).

Specific examples of the MFP-based dye molecules include MFP 590 and MFP 631 (both of which are manufactured by Mobitec Co., Ltd.).

Examples of other dyes include C-phycocyanin, phycocyanin, APC (allophycocyanin), APC-XL and Northern-Lights 637 (manufactured by R&D Systems, Inc.).

In the present invention, from the above-described known fluorescent dye molecules (compounds) or solubilization products thereof, one which satisfies the conditions (1) and (2), preferably one which also satisfies the conditions relating to the Stoke's shift and/or brightness, can be selected for use. Those of ordinary skill in the art should be able to make such selection in accordance with the above-described methods of judging these conditions, without having to bear undue trial and error.

Further, the emission wavelength of the fluorescent dye can be selected as desired in accordance with the intended application. For example, in pathological diagnosis, when such an application where staining with eosin or the like for morphological observation and immunostaining with a fluorescent dye are simultaneously performed is postulated, it is preferred that the fluorescent dye has an emission wavelength in the infrared to near-infrared range so that the light emitted from the fluorescent dye can be visually observed and the emission wavelength of the fluorescent dye does not overlap with that of fluorescence-emitting eosin. For example, a fluorescent dye having its maximum excitation wavelength in a range of 555 to 620 nm and maximum emission wavelength in a range of 580 to 770 nm is preferred.

Specific examples of fluorescent dyes that can be used in the present invention are as follows; however, the present invention is not restricted to embodiments of using these fluorescent dyes.

Examples of fluorescent dyes that can be used with or without being subjected to a solubilization treatment include 5-carboxy-X-rhodamine (5-ROX), Spectrum Red, Alexa Fluor 594, ATTO 590, DY-590, CAL Fluor Red 610 and DyLight 594.

Meanwhile, examples of fluorescent dyes that can be used after being subjected to a solubilization treatment include BODIPY 576/589, PYRROMETHENE 650, Cresyl Violet, DY-610 and Texas Red.

The above-described fluorescent dyes are summarized in Table below.

[Table 1]

| Dye name | Solubilization treatment | Excitation wavelength | Emission wavelengths | Stoke's shift | Skeleton | Molecular weight |
|---|---|---|---|---|---|---|
| BODIPY 576/589 | acetaldehyde | 576 | 590 | 14 | dipyrromethene | 1141.0 |
| 5-ROX | none | 573 | 602 | 29 | rhodamine | 635.8 |
|  | acetaldehyde | 574 | 606 | 32 |  | 678.8 |
| Spectrum Red | none | 587 | 612 | 25 | rhodamine | 490.6 |
|  | concentrated hydrochloric acid | 587 | 612 | 25 |  | 490.6 |
| PYRROMETHENE 650 | acetic acid | 588 | 612 | 24 | dipyrromethene | 301.2 |
| Lumogen F Red 305 | none | 578 | 613 | 35 | condensed polycyclic aromatic system | 1079.2 |
|  | concentrated sulfuric acid | 580 | 618 | 38 |  | 1399.5 |
| BODIPYTR | none | 589 | 617 | 28 | dipyrromethene | 1166.8 |
|  | concentrated sulfuric acid | 589 | 619 | 30 |  | 1247.1 |
| AlexaFluor 594 | none | 590 | 619 | 29 | rhodamine | 758.8 |
|  | formaldehyde | 592 | 621 | 29 |  | 787.8 |
| HiLyte 594 | none | 593 | 618 | 25 | rhodamine | 760.8 |
|  | concentrated hydrochloric acid | 594 | 619 | 25 |  | 760.8 |
| Cresyl Violet | acetaldehyde | 603 | 622 | 20 | oxazine | 339.8 |

(continued)

| Dye name | Solubilization treatment | Excitation wavelength | Emission wavelengths | Stoke's shift | Skeleton | Molecular weight |
|---|---|---|---|---|---|---|
| ATTO 590 | none | 594 | 624 | 30 | rhodamine | 691.2 |
| | formic acid | 595 | 626 | 31 | | 691.2 |
| MFP 590 | none | 597 | 624 | 27 | rhodamine | 691.2 |
| | acetic acid | 597 | 624 | 27 | | 691.2 |
| DY-610 | concentrated sulfuric acid | 610 | 630 | 22 | cyanine | 829.8 |
| Texas Red | formaldehyde | 596 | 615 | 25 | rhodamine | 654.0 |
| DY-590 | none | 580 | 599 | 19 | rhodamine | 719.9 |
| | acetic acid | 580 | 604 | 24 | | 719.9 |
| CAL Fluor Red 610 | none | 587 | 608 | 20 | rhodamine | 919.9 |
| | concentrated hydrochloric acid | 589 | 614 | 25 | | 919.9 |
| DyLight 594 | none | 593 | 618 | 25 | rhodamine | 1078.0 |
| | concentrated sulfuric acid | 593 | 625 | 32 | | 1240.0 |

(Resin)

The resin constituting the resin particle for fluorescent labeling according to the present invention may be a thermosetting resin or a thermoplastic resin. For example, from the standpoint of inhibiting elution of a fluorescent dye in the clearing step using an organic solvent such as xylene, a resin comprising (or consisting of only) a thermosetting resin such as a melamine resin, which is capable of immobilizing a fluorescent dye inside its fine cross-linked structure, is preferred.

Examples of the thermosetting resin include those which contain a structural unit formed from at least one monomer selected from the group consisting of melamine, urea, guanamines (including benzoguanamine, acetoguanamine and the like), phenols (including phenol, cresol, xylenol and the like), xylene and derivatives thereof. Any one of these monomers may be used individually, or two or more thereof may be used in combination. If desired, one or more co-monomers other than the above-described compounds may also be used in combination.

Specific examples of the thermosetting resin include melamine-formaldehyde resins, urea-formaldehyde resins, benzoguanamine-formaldehyde resins, phenol-formaldehyde resins and metaxylene-formaldehyde resins.

As a starting material of these thermosetting resins, in addition to the above-described monomers themselves, a prepolymer obtained by allowing a monomer to react with formaldehyde and other compound such as a cross-linking agent in advance can also be used. For example, in the production of a melamine-formaldehyde resin, generally, methylol melamine prepared by condensation between melamine and formaldehyde under an alkaline condition is used as a prepolymer, and this compound may further be subjected to alkyl-etherification (e.g., methylation for improvement of the stability in water or butylation for improvement of the solubility in an organic solvent).

Further, in the thermosetting resin, at least some of the hydrogens contained in the structural unit may be substituted with a charged substituent or a substituent capable of forming a covalent bond. Such a thermosetting resin can be synthesized by using, as a starting material, a monomer in which at least one hydrogen is substituted with the above-described substituent (derivatized monomer) by a known method. Normally, melamine resins, urea resins, benzoguanamine resins and the like naturally contain an amino group or a cation generated from a moiety originated from an amino group. Also, normally, phenol resins, xylene resins and the like naturally contain a hydroxyl group or an anion generated from a moiety originated from a hydroxyl group.

Such a thermosetting resin can be synthesized in accordance with a known method. For example, a melamine-formaldehyde resin can be synthesized by heating and polycondensing methylol melamine prepared in advance in the above-described manner, with an addition of, as required, a reaction accelerator such as an acid.

Meanwhile, examples of the thermoplastic resin include those which contain a structural unit formed from at least one monofunctional monomer selected from the group consisting of styrene, (meth) acrylic acid, alkyl esters thereof, acrylonitrile and derivatives thereof (a monomer comprising one group involved in polymerization reaction in one molecule, which group is a vinyl group in the above-described case). Any one of these monomers may be used individually, or two or more thereof may be used in combination. If desired, one or more co-monomers other than the above-described compounds may also be used in combination.

Specific examples of the thermosetting resin include styrene-based resins composed of styrene and other monomer(s), such as polystyrenes; acrylic resins composed of (meth)acrylic acid, an alkyl ester thereof and other monomer(s), such as polymethyl methacrylates; and acrylonitrile-based resins composed of acrylonitrile and other monomer(s), such as polyacrylonitriles, AS resins (acrylonitrile-styrene copolymers) and ASA resins (acrylonitrile-styrene-methyl acrylate copolymers).

The above-described thermoplastic resin may also contain, for example, a structural unit formed from a polyfunctional monomer such as divinylbenzene (a monomer comprising two or more groups involved in polymerization reaction in one molecule, which groups are vinyl groups in the above-described case), that is, a cross-linked moiety. Examples of such a thermoplastic resin include cross-linked polymethyl methacrylates.

In the thermoplastic resin, at least some of the hydrogens contained in the structural unit may be substituted with a charged substituent or a substituent capable of forming a covalent bond. Such a thermoplastic resin can be synthesized by using, as a starting material, a monomer in which at least one hydrogen is substituted with the above-described substituent (derivatized monomer), such as 4-aminostyrene.

Further, the thermoplastic resin may also contain a structural unit comprising a functional group used for surface modification of the resulting resin particles for fluorescent labeling. For example, by using an epoxy group-containing monomer such as glycidyl methacrylate as a starting material, resin particles for fluorescent labeling on which epoxy groups are oriented on the surface can be prepared. These epoxy groups can be converted into amino groups by allowing them to react with an excess amount of aqueous ammonia. In the thus formed amino groups, a variety of biomolecules can be incorporated (via, as required, molecules serving as linkers) in accordance with a known method.

-Production Method of Resin Particle for fluorescent labeling-

The resin particle for fluorescent labeling according to the present invention can be produced in accordance with a polymerization step (1) known for various resins, with the use of a fluorescent dye satisfying the specific conditions. Further, the resin particle for fluorescent labeling obtained by such a method may also be ligated with a biologically-relevant binding substance(s) by a modification step (2).

[Thermosetting Resin: Encapsulation-type]

Basically, encapsulation-type resin particles for fluorescent labeling using a thermosetting resin can be produced in accordance with an emulsion polymerization method; however, they are preferably produced by the below-described polymerization step where a surfactant and a polymerization reaction accelerator are used. Here, in the encapsulation-type resin particles for fluorescent labeling that are obtained by such a production method, most of the fluorescent dye, desirably substantially all of the fluorescent dye are immobilized in a state of being encapsulated in the resin particles; however, this does not exclude a mode in which some of the fluorescent dye are immobilized in a state of being bound to or adhered to the surface of the resin particles. Further, in the state where the fluorescent dye is encapsulated, there is no restriction as to by what kind of chemical or physical action the fluorescent dye is immobilized in the resin particles. In the present invention, it is not required to perform, in advance to the polymerization step, a derivatization step for forming covalent bonds between the resin material and the fluorescent dye or actively incorporating a charged substituent into the resin material (resin particles for fluorescent labeling that has excellent emission intensity and light resistance can be obtained even without such a step) ; however, the use of such a step in combination as desired is not excluded either.

(1) Polymerization Step

The polymerization step is a step of generating fluorescent dye-encapsulating resin particles by heating a reaction mixture containing a fluorescent dye and a resin material (a monomer, an oligomer or a prepolymer), and preferably further a surfactant and a polymerization reaction accelerator, and thereby allowing polymerization reaction of the resin to proceed.

The order of adding these components contained in the reaction mixture is not particularly restricted. Typically, an order of adding the surfactant to an aqueous solution of the fluorescent dye, subsequently adding the resin material and then finally adding the polymerization reaction accelerator is employed. Alternatively, it is also possible to employ an order in which the resin material is added to an aqueous solution of the surfactant and then, while allowing the resin particle synthesis reaction to proceed with addition of the polymerization reaction accelerator, an aqueous solution of the fluorescent dye is further added. It is noted here that the concentration of the aqueous solution of the fluorescent dye specified in the present invention, which is used in the above-described polymerization step, can be adjusted to be in a relatively higher range (for example, 250 to 450 $\mu$M) than the concentration of an aqueous solution of a conventional fluorescent dye.

The conditions of the polymerization reaction (e.g., temperature and time) can be set as appropriate taking into consideration the type of the resin, the composition of the material mixture and the like. For the synthesis of a thermosetting resin such as a melamine resin, the reaction temperature is usually 70 to 200°C and the reaction time is usually 20 to 120 minutes. Here, it is appropriate that the reaction temperature be a temperature at which the performance of the fluorescent dye is not reduced (within the range of the heat resistant temperature). The heating can be performed in a plurality of steps and, for example, the material mixture may first be allowed to react for a certain time at a relatively low temperature and then heated and allowed to further react for a certain time at a relatively high temperature.

After the completion of polymerization reaction, impurities such as excess resin material, excess fluorescent dye and excess surfactant are removed from the resulting reaction solution, and the thus generated resin particles for fluorescent labeling can be recovered and purified. For example, after subjecting the reaction solution to centrifugation and removing the resulting supernatant containing impurities, ultrapure water is added to the resin particles and the resultant is ultrasonicated for re-dispersion of the resin particles, followed by washing. It is preferred that these operations be repeated plural times until the resulting supernatant no longer shows any absorption or fluorescence emission attributed to the resin or the fluorescent dye.

(Surfactant)

As the surfactant, a known emulsifier for emulsion polymerization can be used. Examples of the surfactant include anionic, nonionic and cationic surfactants. For the synthesis of a (cationic) thermosetting resin comprising a positively charged substituent or moiety, it is preferred to use an anionic or nonionic surfactant. On the other hand, for the synthesis of a (anionic) thermosetting resin comprising a negatively charged substituent or moiety, it is preferred to use a cationic or nonionic surfactant.

Examples of the anionic surfactant include sodium dodecylbenzenesulfonate (trade name "NEOPELEX" Series, manufactured by Kao Corporation). Examples of the nonionic surfactant include polyoxyethylene alkyl ether-based compounds (trade name "EMULGEN" Series, manufactured by Kao Corporation), polyvinylpyrrolidones (PVPs) and polyvinyl alcohols (PVAs). Examples of the cationic surfactant include dodecyltrimethylammonium bromide.

By adjusting the amount of the surfactant to be added, not only the size of the resulting resin particles can be controlled but also resin particles for fluorescent labeling that have a small variation coefficient of the particle size, that is, uniform particle size, can be produced. The surfactant is added in an amount of, for example, 10 to 60% by weight with respect to the resin material, or 0.1 to 3.0% by weight with respect to the whole material mixture. An increase in the amount of the surfactant tends to reduce the particle size, whereas a decrease in the amount of the surfactant tends to increase the particle size.

(Polymerization Reaction Accelerator)

A polymerization reaction accelerator has functions of not only accelerating polycondensation reaction of a thermosetting resin such as a melamine resin, but also electrically charging a functional group contained in the resin or fluorescent dye, such as an amino group, by donating a proton ($H^+$) thereto to make electrostatic interaction likely to occur. The reaction of a thermosetting resin can be advanced only by heating; however, since an addition of a polymerization reaction accelerator allows the reaction to proceed at a lower temperature, a polymerization reaction accelerator can be added in such a range where it is able to control the reaction and the performance. Examples of the polymerization reaction accelerator include acids such as formic acid, acetic acid, sulfuric acid, p-toluenesulfonic acid and dodecylbenzenesulfonic acid. In cases where the fluorescent dye is a compound having a carboxyl group or a sulfo group, the fluorescent dye is also capable of donating a proton in the same manner as the above-described acids.

(2) Modification Step

The modification step, which is performed as required, is a step of ligating a biologically-relevant binding substance and the like to the surface of the resin particles for fluorescent labeling in accordance with the intended use of the resin particles.

In the technical field to which the present invention belongs, a variety of methods are known for ligating a fluorescent label with a biologically-relevant binding substance and the like. In the present invention as well, any of such methods can be utilized.

For example, by utilizing a reaction that occurs between reactive functional groups such as carboxyl, amino, aldehyde, thiol and maleimide groups, a fluorescent label (a reactive functional group existing on the surface thereof) and a biologically-relevant binding substance (the other reactive functional group existing in the molecule thereof) can be bound with each other. In cases where the functional groups of these substances cannot be directly bound with each other, they can also be bound through a "linker molecule" which has each prescribed functional group at both ends of the molecule. Such reaction can be carried out by adding required reagents and leaving the substances for a prescribed time.

Specific examples of such a method include one in which: an amino group is introduced to resin particles for fluorescent labeling that have a hydroxyl group on the surface by reaction with a silane coupling agent (e.g., aminopropyltrimethoxysilane) while a thiol group is introduced to streptavidin by reaction with a thiol group-introducing agent (e.g., N-succinimidyl-S-acetylthioacetate); and then a PEG (polyethylene glycol)-based linker molecule having a maleimide group, which is reactive with both the amino group and thiol group, at both ends is allowed to react with the resulting resin particles for fluorescent labeling and streptavidin, thereby ligating the resin particles and streptavidin.

Further, in cases where a resin (acrylic resin) is synthesized using, for example, glycidyl methacrylate as a material monomer, epoxy groups originating from the monomer appear on the surface of the resulting resin particles for fluorescent labeling. By adding aqueous ammonia to these resin particles for fluorescent labeling, the epoxy groups are converted into amino groups, and a desired biologically-relevant binding substance and the like can be further bound to the amino groups.

[Thermosetting Resin: Adsorption-type]

The method of producing adsorption-type resin particles for fluorescent labeling using a thermosetting resin is as follows. First, thermosetting resin particles containing no fluorescent dye are prepared in the same manner as in the above-described polymerization step (1) for producing encapsulation-type resin particles for fluorescent labeling, except that a fluorescent dye is not incorporated as a material. Then, the thus obtained resin particles (a dispersion thereof) and a fluorescent dye (an aqueous solution thereof) are mixed, thereby allowing the fluorescent dye to adsorb on the surface of the resin particles. In this case, it is preferred that the thermosetting resin particles and the fluorescent dye each comprise a substituent or moiety having an opposite electric charge and that adsorption therebetween be realized by electrostatic interaction.

[Thermoplastic Resin: Encapsulation-type]

A thermoplastic resin can be synthesized in accordance with a known method such as radical polymerization or ionic polymerization (anionic polymerization or the like). Encapsulation-type resin particles for fluorescent labeling using a thermoplastic resin can also be produced by such method; however, for example, they are preferably produced by a polymerization step in accordance with a soap-free emulsion polymerization method.

In this case, the polymerization step (1) is typically a step of generating fluorescent dye-encapsulating resin particles by heating a reaction mixture containing a fluorescent dye, a resin material and a polymerization initiator (e.g., benzoyl peroxide or azobis-isobutyronitrile) and thereby allowing polymerization reaction of the resin to proceed. The polymerization initiator and the conditions of the polymerization reaction (e.g., temperature and time) can be set as appropriate taking into consideration the type of the resin and the like. For the synthesis of a thermoplastic resin, the reaction temperature is usually 20 to 150°C and the reaction time is usually 10 to 240 minutes.

[Thermoplastic Resin: Adsorption-type]

The method of producing adsorption-type resin particles for fluorescent labeling using a thermoplastic resin is as follows. First, thermoplastic resin particles containing no fluorescent dye are prepared in the same manner as in the above-described polymerization step (1) for producing encapsulation-type resin particles for fluorescent labeling, except that a fluorescent dye is not incorporated as a material. Then, the thus obtained resin particles (a dispersion thereof) and a fluorescent dye (an aqueous solution thereof) are mixed, thereby allowing the fluorescent dye to adsorb on the surface of the resin particles. In this case, it is preferred that the thermoplastic resin particles and the fluorescent dye each comprise a substituent or moiety having an opposite electric charge and that adsorption therebetween be realized by electrostatic interaction.

-Use of Resin Particle for fluorescent labeling-

(Fluorescent Label)

The use of the resin particle for fluorescent labeling according to the present invention is not particularly restricted, and a typical example thereof is the use as a fluorescent label which labels a substance to be detected that is contained in a sample (tissue section) and thereby enables fluorescence observation thereof in immunostaining. That is, the resin particle for fluorescent labeling according to the present invention is preferably bound with a biologically-relevant binding substance conforming to the embodiment of immunostaining and used as a complex (conjugate).

The substance to be detected is not particularly restricted. In pathological diagnosis, generally, an antigen conforming to the purpose thereof is selected. For example, in the pathological diagnosis relating to breast cancer, HER2 can be selected as the substance to be detected.

The first example of the biologically-relevant binding substance is an antibody (primary antibody) which specifically binds to a substance to be detected. A complex composed of the resin particle for fluorescent labeling and a primary antibody is capable of fluorescently labeling a substance to be detected by directly binding thereto (primary antibody method).

The second example of the biologically-relevant binding substance is an antibody (secondary antibody) which binds to an antibody (primary antibody) specifically binding with a substance to be detected. For example, when the primary antibody is an antibody (IgG) produced from a rabbit, the secondary antibody is an anti-rabbit IgG antibody. A complex composed of a fluorescent dye-immobilized resin particle and a secondary antibody is capable of indirectly fluorescently labeling a substance to be detected by binding to a primary antibody bound with the substance to be detected (secondary antibody method).

The third example of the biologically-relevant binding substance includes avidin, streptavidin and biotin. In this case, a secondary antibody forms a complex with a substance capable of binding to a substance forming a complex with the resin particle for fluorescent labeling. For example, when a complex composed of the resin particle for fluorescent labeling and avidin or streptavidin is used, a complex of a secondary antibody and biotin is used in combination. A complex of a secondary antibody and biotin binds to a primary antibody bound with a substance to be detected and, to this complex, a complex composed of a fluorescent dye-immobilized resin particle and avidin or streptavidin further binds, thereby the substance to be detected can be fluorescently labeled indirectly (biotin-avidin method or sandwich method). Conversely, it is also possible to use a complex of the resin particle for fluorescent labeling and biotin in combination with a complex of a secondary antibody and avidin or streptavidin.

As a primary antibody, in accordance with the selected substance to be detected, one which specifically binds thereto can be selected. For example, when the substance to be detected is HER2, an anti-HER2 monoclonal antibody can be used as the primary antibody. Such a primary antibody (monoclonal antibody) can be produced by a conventional method in which a mouse, rabbit, bovine, goat, sheep, dog, chicken or the like is used as an immune animal.

As a secondary antibody, in accordance with the selected primary antibody, one which specifically binds thereto can be selected. For example, when the primary antibody is a rabbit anti-HER2 monoclonal antibody, an anti-rabbit IgG antibody can be used as the secondary antibody. Such a secondary antibody can also be produced by a conventional method.

In addition, when the substance to be detected is a nucleic acid molecule, as a biologically-relevant binding substance corresponding thereto, a nucleic acid molecule having a base sequence complementary to that of the nucleic acid molecule to be detected can be used as well.

A complex in which a biologically-relevant binding substance is bound to the resin particle for fluorescent labeling can be prepared by any known method. For example, amidation by reaction between amine and carboxylic acid, sulfidation by reaction between maleimide and thiol, imination by reaction between aldehyde and amine, or amination by reaction between epoxy and amine can be utilized. The functional groups involved in such reaction may be one which already exists on the surface of the resin particle (a functional group derived from a material monomer of the resin), a functional group existing on the surface of the resin particle that has been converted in accordance with a known method, or a functional group introduced by surface modification or the like. As required, an appropriate linker molecule may also be used.

Accordingly, in another aspect of the present invention, a kit for tissue immunostaining, which comprises the resin particles for fluorescent labeling according to the present invention, is provided. This kit comprises at least: the resin particles for fluorescent labeling according to the present invention; a complex in which a biologically-relevant binding substance is bound to the resin particle for fluorescent labeling, or the resin particle for fluorescent labeling to be used for preparing the complex; the biologically-relevant binding substance; and a reagent(s). As required, this kit may further comprise, for example: a primary antibody; a secondary antibody; other biologically-relevant binding substance (such as biotin) to be used in combination with the above-described biologically-relevant binding substance (such as streptavidin); a reagent(s) used for forming a desired complex; and other reagent(s) used for immunohistological staining.

(Biological Substance Detection Method)

A fluorescent label comprising the resin particle for fluorescent labeling according to the present invention can be used in, for example, a biological substance detection method in which immunostaining and staining for morphological observation are used in combination so as to improve the information property, more specifically, a biological substance detection method which comprises the steps of: (1) immunostaining a tissue section with a fluorescent label; (2) staining the tissue section for morphological observation with a staining agent for morphological observation; and (3) fluorescently observing the stained tissue section. It does not matter whichever of the immunostaining step (1) and the staining for morphological observation step (2) is performed first.

Usually, prior to the step (1) of the biological substance detection method, deparaffinization of the tissue section and activation of the antigen are performed in accordance with conventional methods.

In the immunostaining step (1), required substances can be sequentially added and allowed to react with the tissue section so that the substance to be detected can be properly labeled in accordance with the above-described form of the fluorescent label (a complex of the resin particle for fluorescent labeling and a biologically-relevant binding substance).

The staining for morphological observation step (2) can be performed in accordance with a conventional method. For morphological observation of a tissue specimen, typically, staining with eosin which stains cytoplasm, interstitial tissues, various fibers, erythrocytes and keratinocytes in red to dark red is employed. In addition, staining with hematoxylin which stains cell nuclei, calcareous parts, cartilaginous tissues, bacteria and mucus in livid to light blue is also typically employed (a method in which these two staining techniques are simultaneously performed is known as "hematoxylin-eosin staining" (HE staining)).

Usually, after the steps (1) and (2) but before the step (3), the stained tissue section is subjected to, for example, a dehydration treatment where the tissue section is immersed in ethanol, a clearing treatment where the tissue section is immersed in an organic solvent such as xylene, and a mounting treatment using a mounting medium.

In the fluorescence observation step (3), by irradiating the tissue section subjected to immunostaining and staining for morphological observation in the above-described steps with an excitation light having a wavelength appropriate for the fluorescent dye in use, the fluorescence emitted by the fluorescent label is observed. By this step, a prescribed biomolecule existing in the tissue section, such as an antigen, can be detected, and this information can be utilized to determine the appropriateness of applying a molecular targeted drug (e.g., an antibody pharmaceutical "HERCEPTIN" (trademark), which is a humanized anti-HER2 monoclonal antibody).

For the irradiation of excitation light, the same irradiation means as the one used in ordinary fluorescence observation may be employed. For example, from a laser light source installed in a fluorescence microscope, an excitation light having an appropriate wavelength and output may be irradiated to the stained tissue section using, as required, a filter which selectively allows light having a prescribed wavelength to pass therethrough. Observation of fluorescence may be performed either through the lens barrel of a fluorescence microscope or on a separate display means (e.g., a monitor) showing an image taken by a camera mounted on a fluorescence microscope. Depending on the fluorescent dye, even when the fluorescence cannot be adequately observed visually through the lens barrel of a fluorescence microscope, the fluorescence may be observed on an image taken by a camera in some cases. As required, a filter which selectively allows light having a prescribed wavelength to pass therethrough may also be used.

In the present invention, immunostaining and staining for morphological observation are both performed on the same tissue section. When observing an image produced by the staining for morphological observation, it is not required to irradiate the tissue section with excitation light for exciting the fluorescent dye used in the immunostaining, and the image may be observed under the same observation conditions as those of a light microscope.

EXAMPLES

[Product Information]

- EPOSTAR (registered trademark) (manufactured by Nippon Shokubai Co., Ltd.)

  MX030w: cross-linked polymethyl methacrylate (emulsion concentration: 7.5 to 10%, average particle size: 0.03 to 0.05 $\mu$m)

- NIKALAC (registered trademark) (manufactured by Sanwa Chemical Co., Ltd.)

  MX-035: methylated melamine resin (weight-average polymerization degree: 1.5)
  MS-11: methylated melamine resin (weight-average polymerization degree: 1.8)
  MX-410: methyl/n-butylated melamine resin (weight-average polymerization degree: 1.8)
  MW-022: methylated melamine resin (weight-average polymerization degree: 1.8)
  MX-730: methylated melamine resin (weight-average polymerization degree: 2.4)

- EMULGEN (registered trademark) (manufactured by Kao Corporation)

  EMULGEN 150: polyoxyethylene lauryl ether, amount of effective ingredient: 100%
  EMULGEN 430: polyoxyethylene oleyl ether, amount of effective ingredient: 100%

- NEOPELEX (registered trademark)(manufactured by Kao Corporation)

  NEOPELEX G-15: sodium dodecylbenzenesulfonate, amount of effective ingredient: 16%

- Other

  PVP 30000: polyvinylpyrrolidone (polymerization degree: about 30,000)
  PVA 2000: polyvinyl alcohol (polymerization degree: about 2,000)
  PVA 500: polyvinyl alcohol (polymerization degree: about 500)

[Solubilization Treatment of Fluorescent Dyes]

About 100 mg of each fluorescent dye was prepared and solubilization treatment thereof was performed by adding thereto 100 mL of concentrated sulfuric acid, concentrated hydrochloric acid, acetic acid, formic acid, formaldehyde or acetaldehyde. Then, the thus solubilized fluorescent dye was salted out by adding thereto a saturated NaCl solution and recovered by filtration using a 0.1-$\mu$m filter paper.

[Selection of Fluorescent Dyes]

For the fluorescent dyes shown in Tables below (including solubilized and non-solubilized fluorescent dyes), a 400-$\mu$M aqueous solution (Milli-Q dilution), in which a required amount of each fluorescent dye determined from the molecular weight was dissolved, was prepared, and this aqueous solution was further diluted to prepare 200-$\mu$M, 100-$\mu$M, 90-$\mu$M, 85-$\mu$M, 50-$\mu$M, 75-$\mu$M, 50-$\mu$M, 65-$\mu$M, 60-$\mu$M, 55-$\mu$M, 50-$\mu$M, 45-$\mu$M, 40-$\mu$M, 30-$\mu$M and 20-$\mu$M aqueous solutions. For the thus obtained aqueous fluorescent dye solutions having each concentration, the emission intensity was measured using a fluorophotometer F-7000 manufactured by Hitachi, Ltd. (measurement conditions: photomultiplier voltage = 300 V, excitation light slit = 20 nm, emission slit = 5 nm). Fig. 1 shows the results for five representative fluorescent dyes that were untreated or subjected to the solubilization treatment. From the measurement results, (1) the concentration at peak emission intensity and (2) the ratio of the emission intensity at a concentration of 100 $\mu$M with respect to the peak emission intensity (emission intensity ratio) were determined. The results thereof are shown in Tables below. It is noted here that those fluorescent dyes satisfying both of the conditions (1) and (2) prescribed in the present invention ((1) the peak emission intensity is in the range of 30 to 80 $\mu$M and (2) the emission intensity at a concentration of 100 $\mu$M is not less than 80% of the peak emission intensity) were treated as Examples, and other fluorescent dyes were treated as Comparative Examples.

[Table 2-1]

| | Fluorescent dye | Solubilization treatment | Stoke's shift [nm] | Peak emission intensity | | Emission intensity at concentration of 100 µM [a.u.] (in terms of brightness) | Emission intensity ratio [%] |
|---|---|---|---|---|---|---|---|
| | | | | Concentration [µM] | Emission intensity [au.] (in terms of brightness) | | |
| Comparative Example 1 | BODIPY 576/589 | none | 14 | 65 | 121 (116483) | 82 (78939) | 68 |
| Comparative Example 2 | BODIPY 581/591 | none | 8 | 45 | 106 (102043) | 34 (32731) | 32 |
| Comparative Example 3 | ROX | none | 12 | 35 | 100 (96267) | 42 (40432) | 42 |
| Example 1 | 5-ROX | none | 29 | 55 | 106 (102043) | 89 (85677) | 84 |
| Example 2 | Spectrum Red | none | 25 | 65 | 98 (94341) | 79 (76051) | 81 |
| Comparative Example 4 | PYRROMETHENE 650 | none | 24 | 50 | 95 (91453) | 58 (55835) | 61 |
| Example 3 | Lumogen F Red 305 | none | 35 | 75 | 80 (77013) | 66 (63536) | 83 |
| Comparative Example 5 | CF594 | none | 21 | 35 | 134 (128997) | 75 (72200) | 56 |
| Comparative Example 6 | CAL Flour Red 615 | none | 20 | 45 | 106 (102043) | 60 (57760) | 57 |
| Example 4 | BODIPY TR | none | 28 | 70 | 88 (84715) | 72 (69312) | 82 |
| Example 5 | AlexaFluor594 | none | 29 | 65 | 108 (103968) | 88 (84715) | 81 |
| Example 6 | HilyteFluor 594 | none | 25 | 65 | 102 (98192) | 85 (81827) | 83 |
| Comparative Example 7 | Cresyl Violet | none | 19 | 60 | 45 (43320) | 25 (24067) | 56 |
| Example 7 | ATTO 590 | none | 30 | 65 | 114 (109744) | 95 (91453) | 83 |
| Example 8 | MFP590 | none | 27 | 65 | 122 (117445) | 98 (94341) | 80 |
| Comparative Example 8 | AlexaFluor 610 | none | 16 | 55 | 128 (123221) | 56 (53909) | 44 |
| Comparative Example 9 | DY-610 | none | 20 | 65 | 108 (103968) | 66 (63536) | 61 |

| | Fluorescent dye | Solubilization treatment | Stoke's shift [nm] | Peak emission intensity | | Emission intensity at concentration of 100 μM [a.u.] (in terms of brightness) | Emission intensity ratio [%] |
|---|---|---|---|---|---|---|---|
| | | | | Concentration [μM] | Emission intensity [au.] (in terms of brightness) | | |
| Comparative Example 10 | Cy3.5 | none | 15 | 30 | 123 (118408) | 40 (38507) | 33 |
| Comparative Example 11 | Texas Red | none | 14 | 30 | 120 (115520) | 50 (48133) | 42 |
| Example 9 | DY-590 | none | 19 | 40 | 120 (115520) | 98 (94341) | 82 |
| Example 10 | CAL Flour Red610 | none | 20 | 45 | 123 (118408) | 100 (96267) | 81 |
| Example 11 | Dylight 594 | none | 25 | 65 | 128 (123221) | 119 (114557) | 93 |

[Table 2-2]

| | Fluorescent dye | Solubilization treatment | Stoke's shift [nm] | Peak emission intensity | | Emission intensity at concentration of 100 μM [a.u.] (in terms of brightness) | Emission intensity ratio [%] |
|---|---|---|---|---|---|---|---|
| | | | | Concentration [μM] | Emission intensity [au.] (in terms of brightness) | | |
| Example 12 | BODIPY 576/589 | AA | 14 | 80 | 126 (121296) | 103 (99155) | 82 |
| Comparative Example 12 | BODIPY 581/591 | formic acid | 10 | 55 | 118 (113595) | 50 (48133) | 42 |
| Comparative Example 13 | ROX | concentrated sulfuric acid | 12 | 65 | 122 (117445) | 59 (56797) | 48 |
| Example 13 | 5-ROX | AA | 32 | 75 | 135 (129960) | 116 (111669) | 86 |
| Example 14 | Spectrum Red | concentrated hydrochloric acid | 25 | 75 | 122 (117445) | 105 (101080) | 86 |
| Example 15 | PYRROMETHENE 650 | acetic acid | 24 | 65 | 106 (102043) | 88 (84715) | 83 |
| Example 16 | Lumogen F Red 305 | concentrated sulfuric acid | 38 | 80 | 85 (81827) | 76 (73163) | 89 |
| Comparative Example 14 | CF594 | AA | 23 | 45 | 150 (144400) | 85 (81827) | 57 |
| Comparative Example 15 | CAL Flour Red 615 | acetic acid | 20 | 55 | 122 (117445) | 78 (75088) | 64 |
| Example 17 | BODIPYTR | concentrated sulfuric acid | 30 | 80 | 102 (98192) | 88 (84715) | 86 |
| Example 18 | AlexaFluor 594 | FA | 29 | 75 | 122 (117445) | 105 (101080) | 86 |
| Example 19 | HilyteFluor 594 | concentrated hydrochloric acid | 25 | 70 | 111 (106856) | 96 (92416) | 86 |
| Example 20 | Cresyl Violet | AA | 20 | 80 | 65 (62573) | 55 (52947) | 85 |
| Example 21 | ATTO 590 | formic acid | 31 | 75 | 122 (117445) | 106 (102043) | 87 |
| Example 22 | MFP590 | acetic acid | 27 | 70 | 128 (123221) | 109 (104931) | 85 |

(continued)

| | Fluorescent dye | Solubilization treatment | Stoke's shift [nm] | Peak emission intensity | | Emission intensity at concentration of 100 μM [a.u.] (in terms of brightness) | Emission intensity ratio [%] |
|---|---|---|---|---|---|---|---|
| | | | | Concentration [μM] | Emission intensity [au.] (in terms of brightness) | | |
| Comparative Example 16 | AlexaFluor 610 | FA | 18 | 65 | 132 (127072) | 74 (71237) | 56 |
| Example 23 | DY-610 | concentrated sulfuric acid | 22 | 75 | 122 (117445) | 98 (94341) | 80 |
| Comparative Example 17 | Cy3.5 | formic acid | 15 | 45 | 135 (129960) | 85 (81827) | 63 |
| Example 24 | Texas Red | FA | 25 | 50 | 135 (129960) | 108 (103968) | 80 |
| Example 25 | DY-590 | acetic acid | 24 | 50 | 135 (129960) | 122 (117445) | 90 |
| Example 26 | CAL Flour Red 610 | concentrated hydrochloric acid | 25 | 60 | 138 (132848) | 127 (122259) | 92 |
| Example 27 | Dylight 594 | concentrated sulfuric acid | 32 | 80 | 138 (132848) | 130 (125147) | 94 |

FA: formaldehyde
AA: acetaldehyde

EP 2 966 435 A1

[Synthesis of Fluorescent Dye-immobilized Particles]

The resin particles for fluorescent labeling according to Examples and Comparative Examples were each prepared in accordance with the prescribed conditions shown in Tables below.

[Comparative Examples 1, 10 and 17, and Examples 4, 6, 12, 17 and 19]

Fluorescent Dye-encapsulating Cross-linked PMMA Particle

To 22 mL of water, each of the prescribed fluorescent dyes that were untreated or had been subjected to the solubilization treatment was added in an amount yielding the respective prescribed concentration, and the resultant was heated to 50°C with stirring on a hot stirrer. Then, a predetermined amount of a prescribed cross-linked PMMA material (MX030w) was added thereto, and a predetermined amount of a prescribed polymerization reaction accelerator having a concentration of 10 vol% and the like was further added. The resulting mixture was allowed to react by heating it with stirring at 50°C for 20 minutes, thereby generating fluorescent dye-encapsulating cross-linked PMMA particles. This dispersion was centrifuged (at 20, 000 G for 15 minutes) to recover the particles, which were subsequently purified by washing with ultrapure water.

[Comparative Examples 2 to 4, 6 to 8, 11 to 13, 15 and 16, and Examples 1 to 3, 5, 7 to 11, 13 to 16, 18, 20 to 22, and 24 to 27]

Fluorescent Dye-encapsulating Melamine Resin Particle

To 22 mL of water, a prescribed surfactant was added to a concentration of 0.5 vol%. To this solution, each of the prescribed fluorescent dyes that were untreated or had been subjected to the solubilization treatment was added in an amount yielding the respective prescribed concentration, and the resultant was heated to 70°C with stirring on a hot stirrer. Then, a predetermined amount of a prescribed melamine resin material (e.g., MX-035) was added thereto, and a predetermined amount of a prescribed polymerization reaction accelerator having a concentration of 10 vol% was further added. The resulting mixture was allowed to react by heating it with stirring at 70°C for 50 minutes and then with stirring at 90°C for 20 minutes, thereby generating fluorescent dye-encapsulating melamine resin particles. This dispersion was centrifuged (at 20,000 G for 15 minutes) to recover the particles, which were subsequently purified by washing with ultrapure water.

[Comparative Examples 5, 9 and 14, and Example 23]

Fluorescent Dye-encapsulating Urea Resin Particle

To 22 mL of water, a prescribed surfactant was added to a concentration of 0.5 vol%. To this solution, each of the prescribed fluorescent dyes that were untreated or had been subjected to the solubilization treatment was added in an amount yielding the respective prescribed concentration, and the resultant was heated to 70°C with stirring on a hot stirrer. Then, a predetermined amount of urea was added thereto, and a predetermined amount of formalin having a concentration of 10 vol% was further added. The resulting mixture was allowed to react by heating it with stirring at 70°C for 50 minutes and then with stirring at 90°C for 20 minutes, thereby generating fluorescent dye-encapsulating urea resin particles. This dispersion was centrifuged (at 20, 000 G for 15 minutes) to recover the particles, which were subsequently purified by washing with ultrapure water.

[Table 3-1]

| | Conditions for production of resin particles for fluorescent labeling | | | | | | Evaluation of light resistance | |
|---|---|---|---|---|---|---|---|---|
| | Fluorescent dye | | | Surfactant | Resin material | Polymerization reaction accelerator, etc. | Initial emission intensity [au.] | Relative emission intensity after 10 minutes |
| | Product name | Solubilization treatment | Added amount | | | | | |
| Comparative Example 1 | BODIPY 576/589 | none | 250 μM | none | MX030 0.62 g | AIBN 700 μL | 40 | 45 |
| Comparative Example 2 | BODIPY 581/591 | none | 280 μM | PVA 500 | MX-035 0.65 g | sulfuric acid 650 μL | 20 | 30 |
| Comparative Example 3 | ROX | none | 340 μM | EMULGEN 430 | MS-11 0.6 g | TS 720 μL | 38 | 44 |
| Example 1 | 5-ROX | none | 380 μM | NEOPELEX G-15 | MX-730 0.6 g | DBS 680 μL | 51 | 60 |
| Example 2 | Spectrum Red | none | 320 μM | PVP 30000 | MS-11 0.62 g | formic acid 700 μL | 55 | 61 |
| Comparative Example 4 | PYRROMETHENE 650 | none | 330 μM | EMULGEN 430 | MW-022 0.67 g | TS 620 μL | 40 | 55 |
| Example 3 | Lumogen F Red 305 | none | 380 μM | NEOPELEX G-15 | MX-035 0.70 g | DBS 650 μL | 40 | 61 |
| Comparative Example 5 | CF594 | none | 300 μM | EMULGEN 150 | urea 0.56 g | formalin 600 μL | 60 | 42 |
| Comparative Example 6 | CAL Flour Red 615 | none | 340 μM | PVA 500 | MS-11 0.66 g | DBS 680 μL | 59 | 46 |
| Example 4 | BODIPY TR | none | 380 μM | none | MX030 0.69 g | BPO 620 μL | 39 | 62 |
| Example 5 | AlexaFluor 594 | none | 350 μM | PVA500 | MS-11 0.66 g | formic acid 600 μL | 56 | 60 |
| Example 6 | HilyteFluor 594 | none | 330 μM | none | MX030 0.61 g | BPO 600 μL | 42 | 62 |
| Comparative Example 7 | Cresyl Violet | none | 380 μM | PVP 30000 | MX-730 0.67 g | DBS 600 μL | 21 | 48 |

(continued)

| | Conditions for production of resin particles for fluorescent labeling | | | | | | Evaluation of light resistance | |
|---|---|---|---|---|---|---|---|---|
| | Fluorescent dye | | | Surfactant | Resin material | Polymerization reaction accelerator, etc. | Initial emission intensity [au.] | Relative emission intensity after 10 minutes |
| | Product name | Solubilization treatment | Added amount | | | | | |
| Example 7 | ATTO 590 | none | 280 $\mu$M | PVP 30000 | MX-035 0.65 g | formic acid 640 $\mu$L | 61 | 61 |
| Example 8 | MFP590 | none | 250 $\mu$M | PVA500 | MW-022 0.65 g | formic acid 640 $\mu$L | 64 | 60 |
| Comparative Example 8 | AlexaFluor 610 | none | 290 $\mu$M | EMULGEN 150 | MS-11 0.66 g | TS 600 $\mu$L | 60 | 35 |
| Comparative Example 9 | DY-610 | none | 330 $\mu$M | EMULGEN 430 | urea 0.62 g | formalin 610 $\mu$L | 59 | 50 |
| Comparative Example 10 | Cy3.5 | none | 300 $\mu$M | none | MX030 0.60 g | AIBN 800 $\mu$L | 40 | 45 |
| Comparative Example 11 | Texas Red | none | 150 $\mu$M | NEOPELEX G-15 | MW-022 0.70 g | TS | 50 | 25 |
| Example 9 | DY-590 | none | 350 $\mu$M | PVP 30000 | MS-11 0.65 g | sulfuric acid 800 $\mu$L | 45 | 60 |
| Example 10 | CAL Flour Red 610 | none | 400 $\mu$M | PVA 2000 | MX-730 0.65 g | acetic acid 500 $\mu$L | 48 | 62 |
| Example 11 | Dylight 594 | none | 450 $\mu$M | EMULGEN 430 | MX-035 0.65 g | DBS 500 $\mu$L | 55 | 65 |
| AIBN: azobis-isobutyronitrile BPO: benzoyl peroxide TS: toluenesulfonic acid DBS: dodecylbenzenesulfonic acid | | | | | | | | |

[Table 3-2]

| | Conditions for production of resin particles for fluorescent labeling | | | | | | Evaluation of light resistance | |
|---|---|---|---|---|---|---|---|---|
| | Fluorescent dve | | | Surfactant | Resin material | Polymerization reaction accelerator, etc. | Initial emission intensity [a.u.] | Relative emission intensity after 10 minutes |
| | Product name | Solubilization treatment | Added amount | | | | | |
| Example 12 | BODIPY 576/589 | AA | 280 μM | none | MX030 0.60 g | AIBN 700 μL | 72 | 64 |
| Comparative Example 12 | BODIPY 581/591 | formic acid | 300 μM | PVA 500 | MX-035 0.65 g | sulfuric acid 650 μL | 50 | 37 |
| Comparative Example 13 | ROX | concentrated sulfuric acid | 420 μM | EMULGEN 430 | MS-11 0.6 g | TS 720 μL | 52 | 50 |
| Example 13 | 5-ROX | AA | 400 μM | NEOPELEX G-15 | MX-730 0.6 g | DBS 680 μL | 82 | 72 |
| Example 14 | Spectrum Red | concentrated hydrochloric acid | 380 μM | PVP 30000 | MS-11 0.62 g | formic acid 700 μL | 80 | 72 |
| Example 15 | PYRROMETHENE 650 | acetic acid | 380 μM | EMULGEN 430 | MW-022 0.67 g | TS 620 μL | 75 | 66 |
| Example 16 | Lumogen F Red 305 | concentrated sulfuric acid | 400 μM | NEOPELEX G-15 | MX-035 0.70 g | DBS 650 μL | 72 | 72 |
| Comparative Example 14 | CF594 | AA | 340 μM | EMULGEN 150 | urea 0.56 g | formalin 600 μL | 95 | 50 |
| Comparative Example 15 | CAL Flour Red 615 | acetic acid | 380 μM | PVA500 | MS-11 0.66 g | DBS 680 μL | 71 | 55 |
| Example 17 | BODIPYTR | concentrated sulfuric acid | 400 μM | none | MX030 0.69 g | BPO 620 μL | 70 | 68 |
| Example 18 | AlexaFluor 594 | FA | 370 μM | PVA 500 | MS-11 0.66 g | formic acid 600 μL | 88 | 64 |
| Example 19 | HilyteFluor 594 | concentrated hydrochloric acid | 360 μM | none | MX030 0.61 g | BPO 600 μL | 77 | 68 |

(continued)

| | Conditions for production of resin particles for fluorescent labeling | | | | | | Evaluation of light resistance | |
| | Fluorescent dye | | | Surfactant | Resin material | Polymerization reaction accelerator, etc. | Initial emission intensity [a.u.] | Relative emission intensity after 10 minutes |
| | Product name | Solubilization treatment | Added amount | | | | | |
| Example 20 | Cresyl Violet | AA | 400 µM | PVP 30000 | MX-730 0.67 g | DBS 600 µL | 45 | 63 |
| Example 21 | ATTO 590 | formic acid | 320 µM | PVP 30000 | MX-035 0.65 g | formic acid 640 µL | 81 | 64 |
| Example 22 | MFP590 | acetic acid | 280 µM | PVA500 | MW-022 0.65 g | formic acid 640 µL | 78 | 62 |
| Comparative Example 16 | AlexaFluor 610 | FA | 340 µM | EMULGEN 150 | MS-11 0.66 g | TS 600 µL | 68 | 44 |
| Example 23 | DY-610 | concentrated sulfuric acid | 360 µM | EMULGEN 430 | urea 0.62 g | formalin 610 µL | 77 | 58 |
| Comparative Example 17 | Cy3.5 | formic acid | 350 µM | none | MX030 0.60 g | AIBN 800 µL | 45 | 55 |
| Example 24 | Texas Red | FA | 250 µM | NEOPELEX G-15 | MW-022 0.70 g | TS 500 µL | 71 | 60 |
| Example 25 | DY-590 | acetic acid | 400 µM | PVP 30000 | 0.65 g | sulfuric acid 800 µL | 72 | 62 |
| Example 26 | CAL Flour Red 610 | concentrated hydrochloric acid | 450 µM | PVA 2000 | MX-730 0.65 g | acetic acid 500 µL | 76 | 78 |
| Example 27 | Dylight 594 | concentrated sulfuric acid | 250 µM | EMULGEN 430 | MX-035 0.65 g | DBS 650 µL | 70 | 80 |

FA: formaldehyde
AA: acetaldehyde
AIBN: azobis-isobutyronitrile
BPO: benzoyl peroxide
TS: toluenesulfonic acid
DBS: dodecylbenzenesulfonic acid

[Evaluation of Light Resistance of Resin Particles for fluorescent labeling]

For each of the resin particles for fluorescent labeling that were prepared in Examples and Comparative Examples, the light resistance was evaluated under the observation conditions of a fluorescence microscope, Axio Imager Z2 manufactured by Carl Zeiss AG (excitation wavelength: maximum excitation wavelength - 5 nm; x40, 10 minutes).

First, a 0.1-nM aqueous solution of each resin particle for fluorescent labeling was prepared, and the emission intensity thereof was measured using a fluorophotometer F-7000 manufactured by Hitachi, Ltd. (measurement conditions: photomultiplier voltage = 300 V, excitation light slit = 20 nm, emission slit = 5 nm). Next, this aqueous solution was diluted to 0.01 nM and 5 $\mu$L thereof was sprayed onto an APS-coated glass. After washing the glass with distilled water, Entellan New (manufactured by Merck KGaA), which is a xylene-based mounting medium, was dropped on the glass, and a cover glass was placed thereon to mount the resin particles. This APS-coated glass was set on the stage of the fluorescence microscope, and the fluorescent dye was excited with light having a wavelength of shorter than its maximum excitation wavelength by 5 nm to obtain a fluorescence image (initial emission intensity). After continuing to irradiate the glass with the excitation light for another 10 minutes, a fluorescence image was taken again. The relative value of the emission intensity of this image taken after 10 minutes with respect to the initial emission intensity was calculated. These results are shown under "Evaluation of light resistance" in the above Tables.

**Claims**

1. A resin particle for fluorescent labeling, comprising a fluorescent dye immobilized in a resin particle, said fluorescent dye satisfying both of the following conditions (1) and (2):

   (1) said fluorescent dye alone in an aqueous solution has a concentration-dependent peak emission intensity in a range of 30 to 80 $\mu$M; and
   (2) the emission intensity at a concentration of 100 $\mu$M is not less than 80% of said peak emission intensity.

2. The resin particle for fluorescent labeling according to claim 1, wherein the Stoke's shift of said fluorescent dye in an aqueous solution is not less than 25 nm.

3. The resin particle for fluorescent labeling according to claim 1 or 2, wherein said fluorescent dye has been subjected to a solubilization treatment.

4. The resin particle for fluorescent labeling according to claim 3, wherein said solubilization treatment is an acid treatment.

5. The resin particle for fluorescent labeling according to any one of claims 1 to 4, wherein said fluorescent dye in an aqueous solution has a peak emission intensity of not less than 100,000 in terms of the brightness represented by the following formula:

```
Brightness = Molar extinction coefficient × Quantum yield
× 1/1,000.
```

6. The resin particle for fluorescent labeling according to any one of claims 1 to 5, wherein said fluorescent dye is immobilized in a state of being encapsulated in a resin particle comprising a thermosetting resin.

7. The resin particle for fluorescent labeling according to any one of claims 1 to 6, wherein a biologically-relevant binding substance is ligated to the particle surface.

[Fig. 1]

| | INTERNATIONAL SEARCH REPORT | International application No. |
|---|---|---|
| | | PCT/JP2014/055469 |

**A. CLASSIFICATION OF SUBJECT MATTER**
*G01N21/64*(2006.01)i, *C08J3/14*(2006.01)i, *C08J3/20*(2006.01)i, *C08L101/00*(2006.01)i, *C09B67/02*(2006.01)n

According to International Patent Classification (IPC) or to both national classification and IPC

**B. FIELDS SEARCHED**

Minimum documentation searched (classification system followed by classification symbols)
G01N21/00-21/83, C08J3/14, C08J3/20, C08L101/00, C09B67/02

Documentation searched other than minimum documentation to the extent that such documents are included in the fields searched
Jitsuyo Shinan Koho        1922-1996   Jitsuyo Shinan Toroku Koho   1996-2014
Kokai Jitsuyo Shinan Koho  1971-2014   Toroku Jitsuyo Shinan Koho   1994-2014

Electronic data base consulted during the international search (name of data base and, where practicable, search terms used)
JSTPlus/JMEDPlus/JST7580(JDreamIII)

**C. DOCUMENTS CONSIDERED TO BE RELEVANT**

| Category* | Citation of document, with indication, where appropriate, of the relevant passages | Relevant to claim No. |
|---|---|---|
| Y | WO 2012/029752 A1 (Konica Minolta, Inc.), 08 March 2012 (08.03.2012), paragraphs [0017], [0020] & US 2013/0157287 A1    & EP 2613138 A1 | 1-7 |
| Y | Tetsuo KANO, Masanori JUMONJI, "Optical Output Characteristics of Dye Mixture Laser III -Absorbance and Fluorescence-", The bulletin of Hachinohe Institute of Technology, 2001.02, vol.20, pages 79 to 86 | 1-7 |
| Y | JP 2008-543982 A (Merck Patent GmbH), 04 December 2008 (04.12.2008), paragraphs [0009] to [0018] & US 2008/0193758 A1    & EP 1879934 A & WO 2006/119845 A1    & DE 102005022370 A & KR 10-2008-0015437 A | 3-7 |

|☒| Further documents are listed in the continuation of Box C. | ☐ | See patent family annex. |
|---|---|---|---|

| * | Special categories of cited documents: |
|---|---|
| "A" | document defining the general state of the art which is not considered   to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 15 May, 2014 (15.05.14) | 27 May, 2014 (27.05.14) |

| Name and mailing address of the ISA/ Japanese Patent Office | Authorized officer |
|---|---|
| Facsimile No. | Telephone No. |

Form PCT/ISA/210 (second sheet) (July 2009)

<table>
<tr><td colspan="2" align="center"><b>INTERNATIONAL SEARCH REPORT</b></td><td>International application No.<br><br>PCT/JP2014/055469</td></tr>
</table>

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2010-538298 A  (Carestream Health, Inc.),<br>09 December 2010 (09.12.2010),<br>paragraph [0027]<br>& US 2009/0061532 A1    & US 2007/0238656 A1<br>& EP 2188634 A          & EP 2004723 A<br>& WO 2009/032236 A1     & WO 2007/126834 A2 | 5-7 |
| A | JP 3-029384 A  (Nippon Telegraph and Telephone Corp.),<br>07 February 1991 (07.02.1991),<br>page 6, upper left column, line 20 to upper right column, line 9; table 1; fig. 5<br>(Family: none) | 2 |
| A | JP 3-109509 A  (Toyota Central Research and Development Laboratories, Inc.),<br>09 May 1991 (09.05.1991),<br>page 4, lower right column, line 19 to page 5, upper left column, line 8; fig. 2<br>(Family: none) | 2 |
| A | JP 63-196653 A  (Mitsui Toatsu Chemicals, Inc.),<br>15 August 1988 (15.08.1988),<br>entire text; all drawings<br>(Family: none) | 1-7 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009300334 A
- JP 2012208106 A

- JP 2008543982 W